# EUROPEAN PATENT APPLICATION

(11) **EP 1 389 480 A1**
(43) Date of publication of application: **18.02.2004**
(21) Application number: 02018317.4
(22) Date of filing: 14.08.2002
(51) Int. Cl.: A61P 37/06, A61K 31/167, A61K 31/17, A61K 45/06

(54) **Therapeutical use of guanylhydrazones for the inhibition of CD83 dependent processes and dendritic cell maturation**

(71) Applicant: Mondobiotech Interferon SA, 6925 Gentilino (CH)
(72) Inventor: The designation of the inventor has not yet been filed
(74) Representative: Salgo, Reinhold Caspar, Dr.

(57) **Abstract**

The present invention relates to aromatic guanylhydrazone compounds of the formula 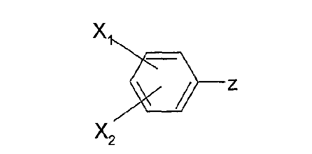 wherein the characters in the formula have the following meanings:
- X₁, X₁', X₁": are - CR¹=N-NH-C(NH)NH₂;
- X₂, X₂', X₂": are -CR²=N-NH-C(NH)NH₂, -H, -OCH₃
- Z: is -H, -NH₂, -CH=CH-COOH,
-C₆H₅ (phenyl), -C₅NH₄ (pyridyl);
-COOB, -OB, -OCOB, -C₆H₄-B, -C₅NH₃-B;
-CR=N-NH-C(NH)NH_{2,} ;
-A-B; -A-C₆H₅, -A-OB,
-A-(CHR)ₙ-B, -A-(CHR)ₙ- A'-B,
-A-(CHR)ₙ-Q-(CHR')ₘ-B, -A-(CHR)ₙ-Q-(CHR')ₘ- A'-B
- A, A', A": are -NHCO-, -CONH-, -NH;-
- R, R¹, R², R', R": are -H, C₁ - C₃- alkyl;
- B: is 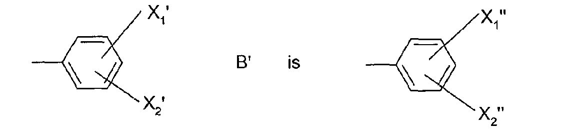
- Q: is 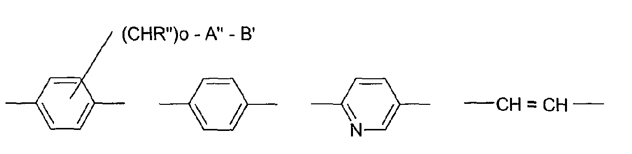
- n, m, o: are independently from each other integers from 0 to 10,
and their use as pharmaceutically active agents as inhibitors of expression of CD83 molecules and maturation of dendritic cells. They are especially useful in organ transplantation.

## Description

### TECHNICAL FIELD OF THE INVENTION

The present invention relates to aromatic guanylhydrazone compounds and their use as pharmaceutically active agents as inhibitors of expression of CD83 molecules on dendritic cells and may be used to inhibit the maturation of immunocompetent dendritic cells. Especially the compounds can be used for prophylaxis and treatment of organ transplantations. In addition, methods for treating and supporting successful organ transplantations are disclosed together with pharmaceutical compositions useful within said methods.

### BACKGROUND OF THE INVENTION

The present invention relates to the use of compounds of the general formula (I): 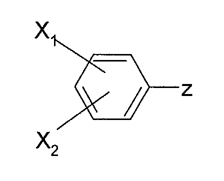 and the characters in the formula have the following meanings:
- X₁, X₁', X₁" are: - CR¹=N-NH-C(NH)NH₂;
- X₂, X₂', X₂" are: -CR²=N-NH-C(NH)NH₂, -H, -OCH₃
- Z is: -H, -NH₂, -CH=CH-COOH, -C₆H₅ (phenyl), -C₅NH₄ (pyridyl); -COOB, -OB, -OCOB, -C₆H₄-B, -C₅NH₃-B; -CR=N-NH-C(NH)NH₂, ; -A-B; -A-C₆H₅, -A-OB, -A-(CHR)ₙ-B, -A-(CHR)ₙ- A'-B, -A-(CHR)ₙ-Q-(CHR')ₘ-B, -A-(CHR)ₙ-Q-(CHR')ₘ- A'-B
- A, A', A" are: -NHCO-, -CONH-, -NH;-
- R,R¹,R²,R',R" are: -H, C₁- C₃- alkyl;
- B is: 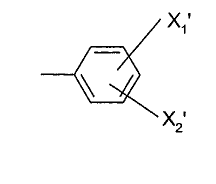 B' is 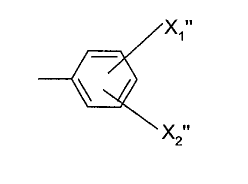
- Q is: 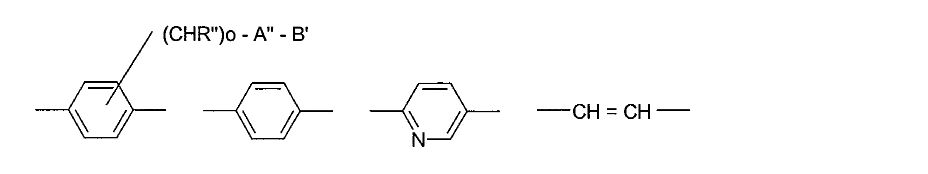
- n, m, o are: independently from each other integers from 0 to 10.

Compounds having the general formula (I) wherein A = A' are known from U.S. patents 5,599,984; 5,750,573; 5,753,684 as inhibitors of the uptake of arginine by macrophages and/or its conversion to urea. Therefore, these compounds can be used for treating arginine-dependent tumors and infections. Further, the suitability of these compounds in preventing the generation of nitric oxide (NO) by cells and to prevent NO-mediated inflammation and other responses is disclosed. WO98/20868 discloses a method for treating diseases and disorders involving T-cell activation and HIV infection using the p38 mitogen activated protein kinase (MAPK) signaling pathway as target for the intervention. WO01/56553A2 discloses methods for prophylaxis and treatment of virally caused diseases and infections, including opportunistic infections. It is suggested that guanylhydrazone compounds according to formula (I) act as MAPK inhibitors. U.S. 5,849,794 discloses the use of some compounds of the general formula (I) wherein A = A' for preventing and ameliorating cachexia, the clinical syndrome of poor nutritional status and bodily wasting associated with cancer and other chronic diseases, while U.S. 5,854,289 describes the use of said compounds for treating multiple sclerosis (autoimmune encephalomyelitis) and U.S. 6,143,728 discloses their use for treating a disorder or disease characterized by T cell activation. U.S. 2,002,028,851 describes the use of said compounds for inhibition of arginine uptake in arginine-dependent tumors and infections. Other compounds of the general formula (I), in which Z bears the residue Q and Q is a phenyl residue substituted with the above-defined - ( CHR")ₒ - A" - B' residue, are known from U.S. 5,859,062 and 5,849,794 wherein the use of such compounds for treating inflammatory diseases mediated by TNF and a method for treatment of neoplastic diseases are disclosed, as well as from WO 01/56553 where those compounds are disclosed for prophylaxis and treatment of virally caused diseases and infections, including opportunistic infections. Furthermore, compounds of the general formulas (I) are described in U.S. 6,022,900 and 6,008,255 together with their use for treating whole body nitrogen loss associated with catabolic illness or for treating endotoxic shock in a subject.

Dendritic cells (DC) are considered to be the most potent antigen-presenting cells, and CD83 is expressed at a high level on immune-competent, activated and mature DC. CD83 is a 45-kDa glycoprotein and member of the immunoglobulin (Ig) superfamily. It is the best known marker for mature dendritic cells. Although the precise function of CD83 is not known, its selective expression and upregulation together with the costimulators CD80 and CD86 suggests an important role of CD83 in the induction of immune responses.

DC are potent antigen presenting cells that possess the unique ability to stimulate naive T-cells and are crucial in the induction of immunological responses. DC based immunotherapies are thus currently considered a particularly promising approach for cellular immunotherapy.
By studying DC derived from various tissues it has been shown that the morphology, phenotype and function of DC alter as they undergo a complex process of maturation. DC are derived from bone marrow progenitors and circulate in the blood as immature precursors prior to migration into the peripheral tissues. Within tissues DC are specialised in the taking up and processing of antigen so that it may be presented on MHC class II molecules. Upon appropriate stimulation, tissue DC undergo further maturation and migrate to secondary lymphoid tissue where they present antigen to T-cells and induce an immune response. Studies of DC maturation in vitro have defined the cytokines regulating their development from CD34+ myelomonocytic progenitors as well as from more mature peripheral blood precursors. An alternative pathway of differentiation from thymic precursors has also been described. As a result of these studies, DC may now be generated and manipulated ex-vivo for clinical applications in oncology, autoimmune disease and transplantation.
Following enrichment, DC develop an activated phenotype with up-regulation of CD80, CD86, and CD83 expression.
CD83 is a functionally important receptor that can regulate the development of cellular immunity by interacting with its ligand(s).
Immature DCs capture antigens in the periphery but lack full T cell-stimulatory capacity. In the presence of appropriate stimuli (such as microbial products and/or inflammatory cytokines), the DCs then mature. DCs upregulate T cell adhesion and costimulatory molecules as well as selected chemokine receptors that guide DC migration into secondary lymphoid organs for priming of antigen-specific T cells. DCs are defined by their potent T cell-stimulatory capacity (e.g., in the allo-MLR) as well as a characteristic morphology (nonadherent cells with motile veils) and phenotype (upregulation of CD86 and de novo expression of CD83).
DC can be generated in vitro either from rare proliferating CD34⁺ or frequent, nonproliferating CD14⁺ monocytic precursors. The generation of DCs from monocytes under the use of GM-CSF and IL-4 yields homogenous DC progenitors that are well suited for studying the maturation of DC in vitro.
Functional, mature DC are derived from circulating precursor cells after a period of maturation and express then a specific array of marker molecules. These include the accessory / costimulatory gene products CD40, CD80, and CD86 as well as MHC class I and II. In
particular, the presence of the CD83 molecule is a well characterized marker for fully mature DC, as CD83 can not be detected on immature DC precursors.
It is known from literature that formation of hypusine on the eukaryotic initiation factor 5A (eIF-5A) protein is required for the expression of the DC-specific molecule CD83 and the full stimulatory activity of mature DC. The hypusine formation is required for efficient nuclear export of CD83 mRNA. Thus, CD83 mRNA exploits a specific pathway for its transport from the nuclear site of RNA transcription and processing to the site of translation in the cytoplasm. The eIF-5A protein has an unique biochemical feature in that it is the only eukaryotic cellular protein known containing the unusual amino acid hypusine. Hypusine formation is a modification of the lysine residue at amino acid position 50 within the 154 amino acids containing eIF-5A precursor molecule by a two-step posttranslational enzymatic reaction. In the first step, the cellular enzyme deoxyhypusine synthase (DHS) catalyzes the transfer of an aminobutyl-moiety from spermidine to the lysine 50 within eIF-5A via an enzyme-substrate intermediate formation at lysine 329 of the DHS to generate the eIF-5A deoxyhypusine form. This eIF-5A deoxyhypusine intermediate is subsequently hydroxylated by the enzyme deoxyhypusine hydroxylase, resulting in biologically active, hypusine-containing eIF-5A. Hypusine formation is essential for the biological activity of the eIF-5A protein. In addition, biologically active eIF-5A protein is essential for nuclear export of Rev proteins and, thereby, HIV replication, for the function of the Human T-cell leukemia virus type I (HTLV-I) Rex protein which is itself crucial for HTLV-I replication, as well as for the cell surface expression of the CD83 molecule which is crucially involved in the maturation of dendritic cells.
In general, the translocation of mRNAs across the nuclear pore requires a protein carrier, which is part of a complex system located in the nuclear envelope. In this view, eIF-5A would be a part of a transport system which is involved in the translocation of nuclear RNA to the cytoplasm. After transit through the nuclear pores and assembly of translation-competent ribosomes, the preferentially translocated mRNAs would be utilized in the process of protein synthesis. Therefore, small molecular weight chemical substances that interfere with the biological function of the eIF-5A protein at the level of nuclear transport are potential compounds by interfering with nucleocytoplasmic translocation of mRNAs, especially in the field of organ transplantations.

### SUMMARY OF THE INVENTION

The present invention relates to the use of compounds of the general formula (I): 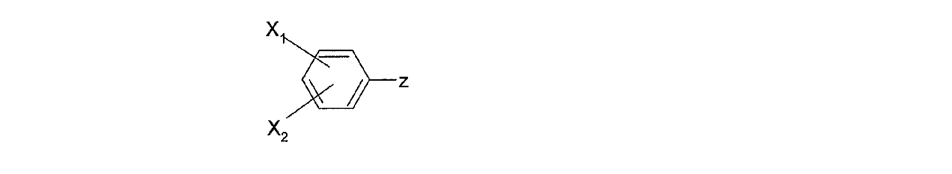 and the characters in the formula have the following meanings:
- X₁, X₁', X₁" are: - CR¹=N-NH-C(NH)NH₂;
- X₂, X₂', X₂" are: -CR²=N-NH-C(NH)NH₂, -H, -OCH₃
- Z is: -H, -NH₂, -CH=CH-COOH, -C₆H₅ (phenyl), -C₅NH₄ (pyridyl); -COOB, -OB, -OCOB, -C₆H₄-B, -C₅NH₃-B; -CR=N-NH-C(NH)NH₂, ; -A-B; -A-C₆H₅, -A-OB, -A-(CHR)ₙ-B, -A-(CHR)ₙ- A'-B, -A-(CHR)ₙ-Q-(CHR')ₘ-B, -A-(CHR)n-Q-(CHR')m- A'-B
- A, A', A" are: -NHCO-, -CONH-, -NH;-
- R, R¹, R², R', R" are: -H, C₁ - C₃- alkyl;
- B is: 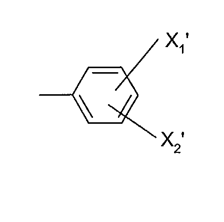 B' is 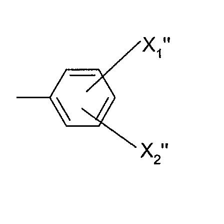
- Q is: 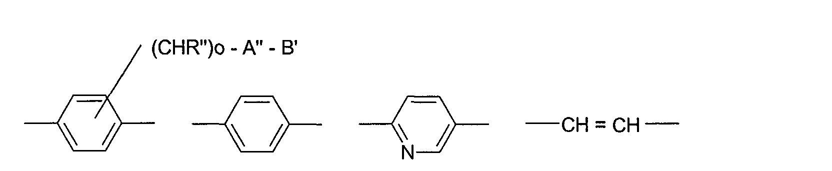
- n, m, o: are independently from each other integers from 0 to 10.

Surprisingly, the above-specified guanylhydrazone compounds of the present invention including their pharmaceutically acceptable salts exhibit excellent activity against expression of CD83 molecules on dendritic cells and inhibit maturation of dendritic cells.

Furthermore, compounds of the general formula (I) are preferred wherein Z represents A-(CHR)ₙ-B, -A-(CHR)ₙ- A'-B, -A-(CHR)ₙ-Q-(CHR')ₘ-B, -A-(CHR)n-Q-(CHR')m- A'-B and n and m is an integer of 1 to 10, preferably 1 - 5.
More preferably Z represents
-A-(CH₂)ₙ-A'-B or
-A-(CH₂)ₙ-B,
-A-(CH₂)ₙCH=CH-(CH₂)ₘ-A'-B,
-A-(CH₂)ₙ-CH=CH-(CH₂)ₘ-B,
-A-(CH₂)ₙ-C₆H₄-(CH₂)ₘ-A'-B,
-A-(CH₂)ₙ-C₆H₄-(CH₂)ₚ-B,
-A-(CH₂)ₙCR'R''-(CH₂)ₘ-A'-B,
-A-(CH₂)ₙ-CR'R"-(CH₂)ₘ-B,
-A-(CH₂)ₙ-C₅H₃N-(CH₂)ₙ,-A'-B, or
-A-(CH₂)ₙC₅H₃N-(CH₂)ₘ-B and
n and m are independently of each other integer of 1 to 5, preferably 1, 2 or 3.

Furthermore the following sub-formulas (II - VIII), wherein the characters in the formulas have the meaning as indicated above, are preferred:

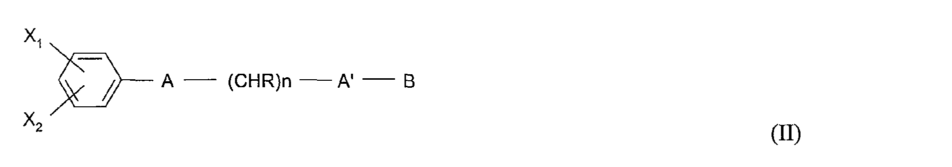

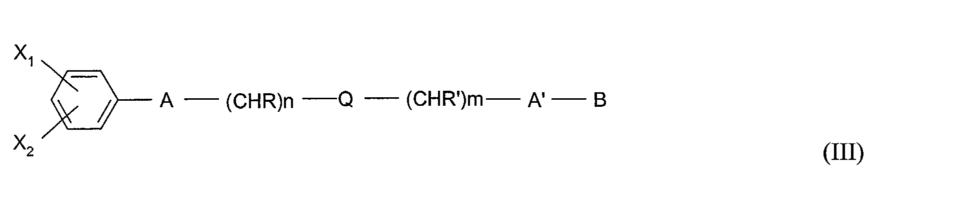

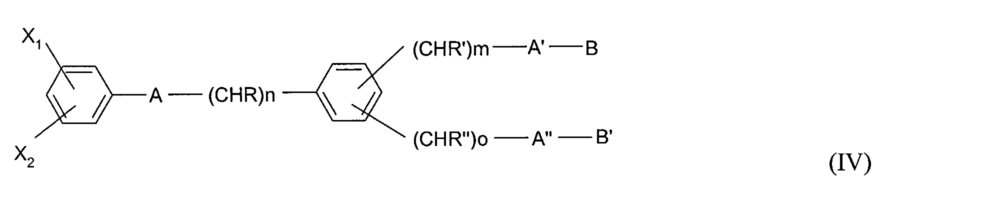

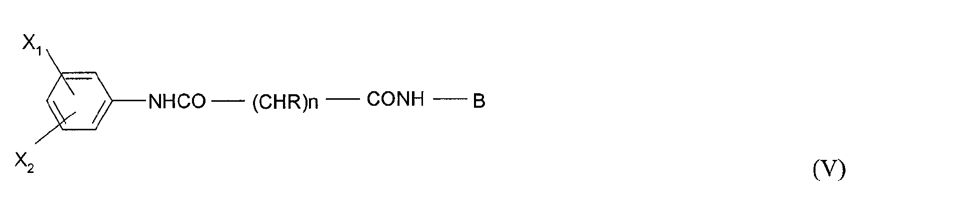

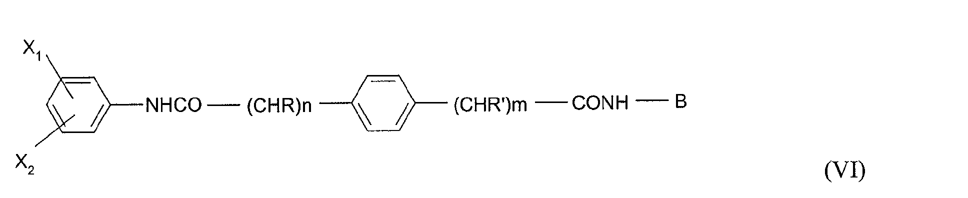

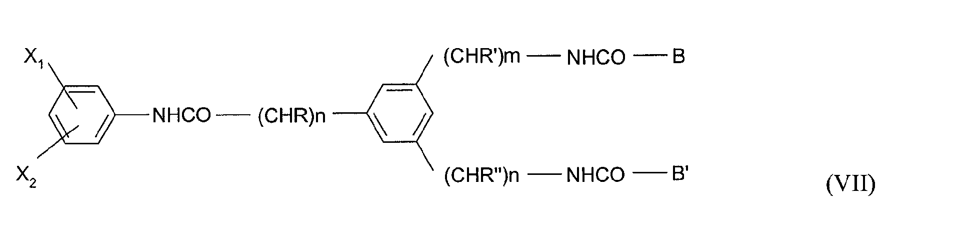

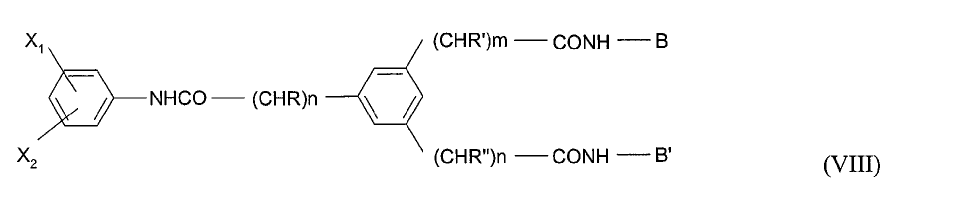

The substituents other than hydrogen in each phenyl residue of the compounds of the formula (I) - (VIII) are preferably in para-position if only two of said substituents exist in each phenyl residue. If three substituents other than hydrogen are present in a phenyl residue these substituents are preferably in meta-position to each other. The meta-position is especially preferred if large-sized substituents are used because of steric reasons, which is above all the case for compounds of formulas (IV), (VII) and (VIII).

Most preferred is the use of the inventive compounds selected from the group comprising or salts thereof:

### N-(4-acetylphenyl)-N'-(3,5-diacetylphenyl)urea tris (amidinohydrazone):

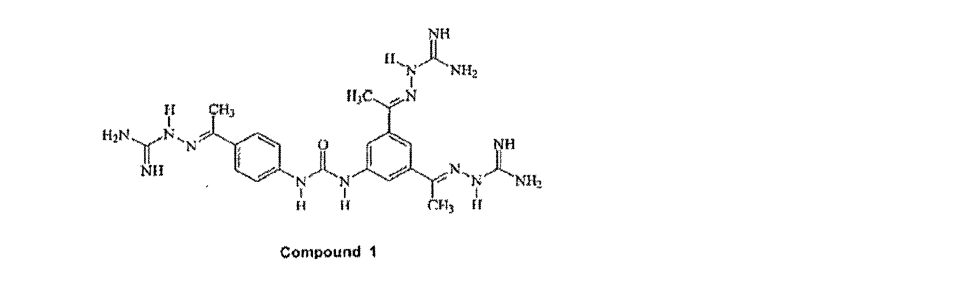

### N,N'-bis (3-acetylphenyl) pentane diamide bis (amidinohydrazone):

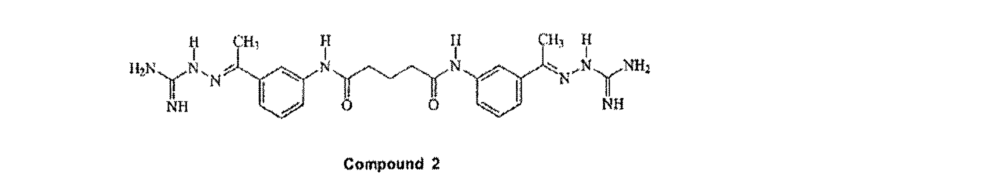

### N,N'-bis (3,5-diacetylphenyl) pentane diamide tetrakis (amidinohydrazone):

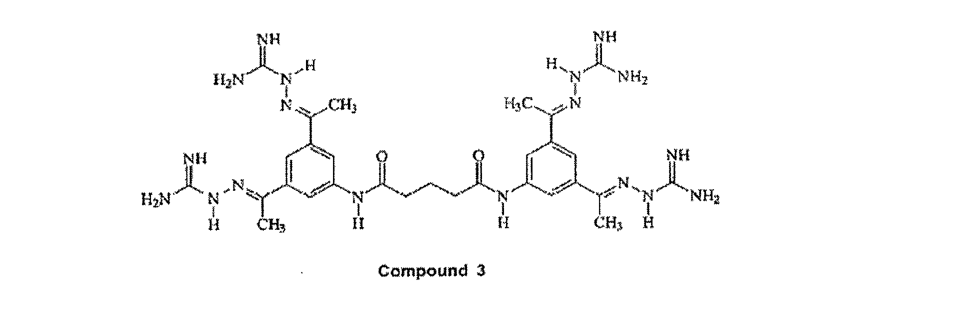

### N,N'-bis (3,5-diacetylphenyl) decane diamide tetrakis (amidinohydrazone):

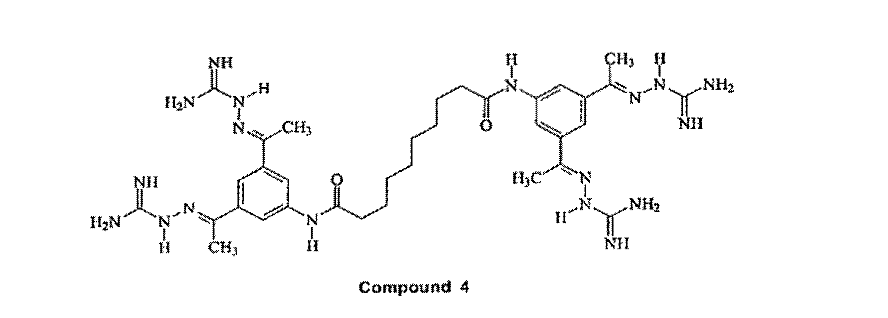

### N,N'-bis (3,5-diacetylphenyl) butane diamide tetrakis (amidinohydrazone):

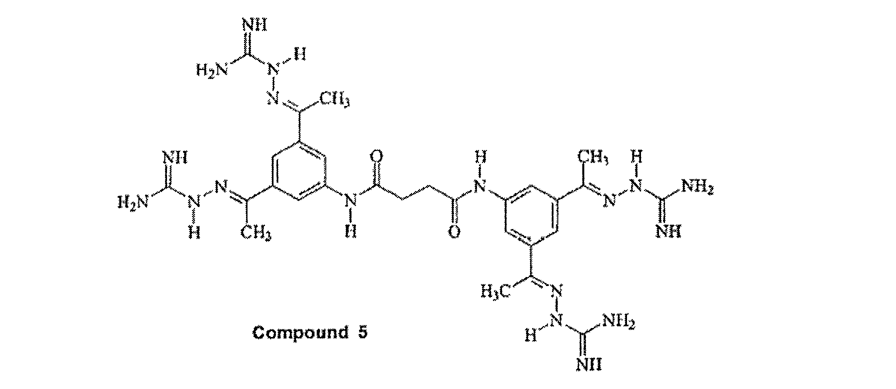

### N,N'-bis (3,5-diacetylphenyl) hexane diamide tetrakis (amidinohydrazone):

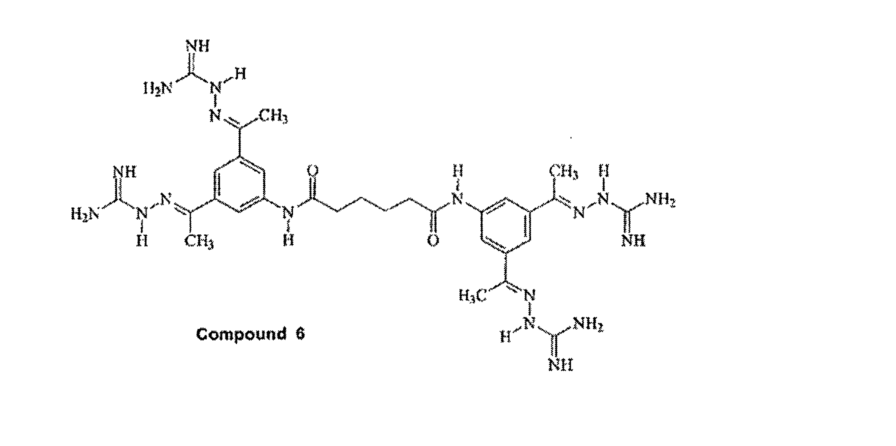

### N,N'-bis (3,5-diacetylphenyl) heptane diamide tetrakis (amidinohydrazone):

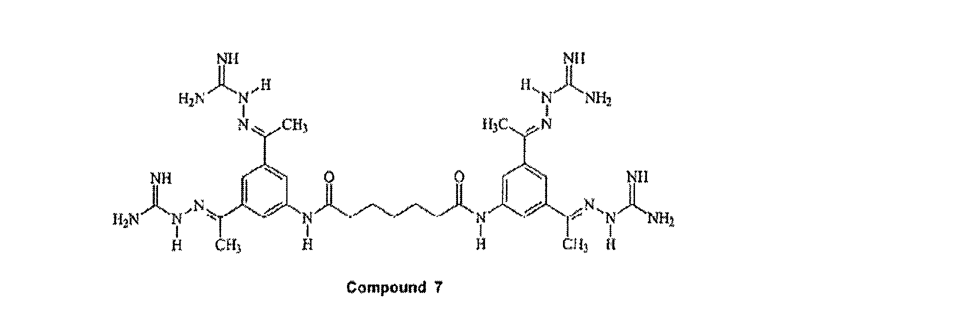

### N,N'-bis (3,5-diacetylphenyl) isophthalic acid diamide tetrakis (amidiho-hydrazone):

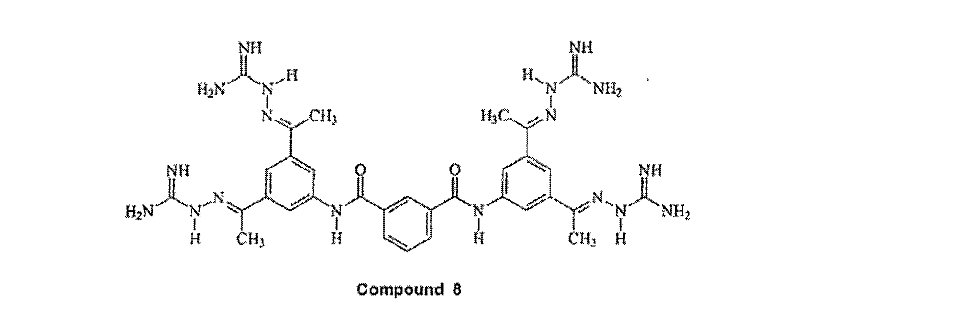

To sum up, the invention relates to the following topics:
- Use of a compound of the formula (I) - (VIII) or a pharmaceutically acceptable salt thereof for the manufacture of a medicament for the inhibition of a CD83 functionally dependent process in an individual, wherein the formula (I) - (VIII) are defined above.
- A corresponding use, wherein compounds are selected where A and A' are different.
- A corresponding use, wherein said inhibition is directed to the nucleocytoplasmic translocation of CD83 mRNA molecules.
- A corresponding use, wherein said inhibition is directed to the expression of CD83 protein.
- A corresponding use, wherein the expression of CD83 on dentritic cells is inhibited.
- A corresponding use, wherein said inhibition is directed to the maturation of dentritic cells.
- A corresponding use, for the treatment and / or prevention of graft rejection during or following organ transplantation.
- A corresponding use, wherein the organ suitable for said transplantation is selected from the group: kidney, liver, heart, pancreas, lung and bone marrow.
- A corresponding use, wherein said organ transplantation comprises treatment of cells of said organ *ex vivo* with any of the compounds of the formula I - VIII.
- A corresponding use, wherein said cells are the complete or parts of the bone marrow cells of said individual.
- A corresponding use, wherein the effective dosage of any of said compounds corresponds to a concentration in the range of 0.01 - 10 uM, preferably 0.1 - 5 uM.
- A corresponding use, wherein said treatment is achieved in combination with further medicaments used in or after organ transplantation.
- A corresponding use, wherein the effective dosage of said further medicament is lower than when administered alone in monotherapy.
- A corresponding use, wherein said further medicament is selected from the group consisting of: mycophenolat mofetil, brequinar, cyclosporine, tacrolimus, sirolimus, spergualin, FTY720.
- A pharmaceutical composition comprising one or more compounds of the formulas I to VIII or a pharmaceutically acceptable salt thereof, a further different medicament suitable in or after organ transplantation, and, optionally, a pharmaceutically acceptable carrier, excipient or diluent.
   A corresponding pharmaceutical composition, wherein said further different medicament is selected from the group consisting of: mycophenolat mofetil, brequinar, cyclosporine, tacrolimus, sirolimus, spergualin, FTY720.
- A pharmaceutical kit comprising a first package containing a compound of any of the formulas I to VIII, as defined in any of the preceding claims, and a second package containing a compound selected from the group consisting of: mycophenolat mofetil, brequinar, cyclosporine, tacrolimus, sirolimus, spergualin, FTY720.
- A Method of inhibiting of a CD83 dependent process in an individual comprising administering to said individual in an effective dosage a compound of formula I or one of its sub-formulas II to VIII, wherein the formulas are defined above and in the claims.
- A corresponding method, wherein compounds are selected where A and A' are different.
- A corresponding method, wherein said inhibition is directed to the nucleocytoplasmic translocation of CD83 mRNA molecules.
- A corresponding method, wherein said inhibition is directed to the expression of CD83 protein.
- A corresponding method, wherein the expression of CD83 on dentritic cells is inhibited.
- A corresponding method, wherein said inhibition is directed to the maturation of dentritic cells.
- A corresponding method for the treatment and / or prevention of graft rejection during or following organ transplantation comprising administering to an individual in an effective dosage a compound of any of the formulas I to VIII, as defined above and in the claims.
- A corresponding method for the treatment and / or prevention of graft rejection during or following organ transplantation of an individual, comprising isolating diseased celles from said organ and treating them ex vivo with an effective amount of a compound of the formulas I to VIII.
- A corresponding method, wherein said diseased cells are the complete or parts of the bone marrow cells of said individual.
- A corresponding method, wherein said effective dosage of the compound corresponds to a concentration in the range of 0.01 - 10 uM, preferably 0.1 - 5 uM.
- A corresponding method, wherein a further medicament is administered to the individual which is used in or after organ transplantation and is different from those compounds as specified in any of the formulas I to VIII.
- A corresponding method, wherein sad further medicament is administered in an effective dosage which is lower than when administered alone in a monotherapy.
- A corresponding method, wherein said further medicament is selected from the group consisting of: mycophenolat mofetil, brequinar, cyclosporine, tacrolimus, sirolimus, spergualin, FTY720.

### DETAILED DESCRIPTION OF THE INVENTION

One aspect of the present invention is directed to the use of a compound of the general formula (I) and/or (II) and/or pharmaceutically acceptable salts thereof as an inhibitor of nuclear transport or export of messenger RNAs of proteins involved in the biology of organ rejections.
Surprisingly, it was found that the guanylhydrazone compounds according to this invention exhibit excellent activity against maturation of dendritic cells, a feature which is highly beneficial in organ transplantation.

The compounds of the present invention and/or pharmaceutically active salts thereof can be used for the manufacture of an agent for prophylaxis and/or treatment of graft rejection following organ transplantation. Examples for such organ transplantations comprise kidney, liver, heart, heart/lung, pancreas, corneas, bone marrow, skin, bone, heart valves, lung, intestine transplantations.
Surprisingly it was found that the aromatic guanylhydrazones and pharmaceutically acceptable salts thereof are potent inhibitors of eIF-5A dependent nuclear transport of CD83 mRNA molecules. The inhibition of the nuclear transport represents a novel target for prevention or treatment of graft rejection in organ transplantations.
Testing of compounds of the general formulas (I) - (VIII) in relevant primary human dendritic cell systems revealed that these compounds are potent inhibitors of nucleocytoplasmic translocation of CD83 mRNA in the submicromolar and low micromolar range. Thus another aspect of the present invention is directed to the use of the compounds according to this invention and/or pharmaceutically active salts as inhibitors of CD83 protein expression and dendritic cell maturation.
The compounds of the present invention act as inhibitors of dendritic cell maturation. Thus, a further aspect of the present invention is related to a method for inhibiting maturation of dendritic cells for the prevention or treatment of graft rejections in organ transplantations, comprising administering a subject in need thereof a pharmaceutically effective amount of at least one compound of the general formulas (I) to (VIII) and/or pharmaceutically active salts thereof.

Since eIF-5A protein is involved in the nuclear transport, the compounds of the present invention act as modulators of nuclear transport, particularly nuclear transport of RNA molecules. More particularly, the compounds of the inventions may be used for the manufacture of an agent capable of inhibiting the export of CD83 RNA molecules from the nucleus to the cytoplasm of a person in need for organ transplantation. Particularly, the present invention provides an agent and a method for the prevention or treatment of graft rejections in organ transplantations.

Thereby, the aromatic guanylhydrazones and/or pharmaceutically acceptable salts thereof are administered in an ex-vivo dosage corresponding to an effective concentration in the range of 0.01-10 µM. More preferably, the compounds of the present invention are administered in a dosage corresponding to an effective concentration in the range of 0.1-5 µM.

Surprisingly, it was found that the compounds of formulas (I) to (VIII) are potent inhibitors of dendritic cell maturation in a submillimolar, especially submicromolar, range on without apparent cellular toxicity at the effective biological anti CD83 expression dosage.
These findings suggest that the mentioned compound class of aromatic guanylhydrazones may have advantages over the currently used drugs like mycophenolate mofetil (RS-61443; Syntex, San Jose, CA, USA), brequinar (DUP785; DuPont-Merck, Wilmington, DE, USA), cyclosporine (Novartis, Basle, Switzerland), tacrolimus (FK506; Fujisawa, Osaka, Japan), sirolimus (rapamycin or rapamune; Wyeth-Ayerst, Princeton, NJ, USA), spergualin (1-amino-19-guanitido-11,15-dihydroxy-4,9, 12-triazathioprinenonadecane- 10,13-dione; Nippon Kayaku Co., Tokyo, Japan), FTY720 (Novartis, Basle, Switzerland), in the treatment of organ transplantations, e.g. Kidney, Liver, Heart, Heart/Lung, Pancreas, Corneas, Bone Marrow, Skin, Bone, Heart Valves, Lung, Intestine transplantations.

Those therapeutic agents are accompanied by severe side effects. Cyclosporine and tacrolimus display overlapping nephrotoxicities, whereas sirolimus produces hypertriglyceridaemia. Spergualin, another T-cell-selective inhibitor, is of limited use for transplantation owing to its low bioavailability and considerable toxicities. The in vivo effects in humans and the mechanism of action of the new agent FTY720 are as yet unknown. Recently, more-selective inhibitors of nucleotide synthesis have been developed, including mycophenolate mofetiland brequinar; these agents constitute the second generation of antiproliferative immunosuppressive drugs. However, although mycophenolate mofetil significantly decreases the incidence of rejections in recipients of kidney allografts, it appears to have no effect on long-term graft survival rates, whereas brequinar is significantly toxic.

In order to treat said complications related to graft rejections in organ transplantations at least one compound of the general formulas (I) to (VIII) and/or pharmaceutically effective salts thereof are administered to an individual in need according to the disclosed method in a dosage corresponding to an effective concentration in the range of 0.01-10 µM, more preferably in the range of 0.1-10 µM, most preferably 0.1-5 µM.
Furthermore, the aromatic guanylhydrazone compounds may be administered directly or in combination with further therapeutic compounds, especially with further agents used in organ transplantation.
A list of suitable agents comprises the compounds: mycophenolate mofetil, brequinar, cyclosporine, tacrolimus, sirolimus, spergualin, FTY720.

Following is a comparison of the USA numbers of organ transplants done in 2000 and the numbers of individuals who remained on the national waiting list as of February 2002.

| **Organ** | **Number of Transplants** |
|---|---|
| Kidney | 13,372 |
| Kidney/Pancreas | 911 |
| Pancreas | 435 |
| Liver | 4,954 |
| Heart | 2,198 |
| Heart / Lung | 48 |
| Lung | 956 |
| Intestine | 79 |
| Total | 22,953 |

In relation to the above statements, the use of the aromatic guanylhydrazone compound of the present invention and a method for the use of the compounds and/or pharmaceutically active salts of said compounds is disclosed wherein at least one compound of the general formula (I) and/or (II) and/or pharmaceutically active salts thereof is administered in combination with further therapeutic compounds, especially with further agents used in organ transplantations. Said further agents may be selected from the following drugs: mycophenolate mofetil, brequinar, cyclosporine, tacrolimus, sirolimus, spergualin, FTY720.

Thus, the compounds described in the present invention can be used in a monotherapy directly or in form of pharmaceutically acceptable compositions in order to treat graft rejection in organ transplantations.

The compounds described in the present invention can be specifically combined with mycophenolate mofetil, brequinar, cyclosporine, tacrolimus, sirolimus, spergualin, FTY720. In these cases the effective dosage of said further medicament may be 5 - 25% lower than when administered alone in monotherapy showing the same or a comparable activity.
This invention also relates to the combination of the compounds described in the present invention with at least one of the above mentioned drugs.

The aromatic guanylhydrazone compounds of the present invention or pharmaceutically effective salts thereof are preferably administered in a dosage corresponding to an effective concentration in the range of 0.01-10 µM, more preferably in the range of 0.1-10 µM, and most preferably in the range of 0.1-5 µM.

As mentioned above, a preferred embodiment of the present invention relates to the use of the aromatic guanylhydrazone compounds of the general formulas (I) to (VIII) in combination with further agents used in organ transplantations. Mycophenolate mofetil, brequinar, cyclosporine, tacrolimus, sirolimus, spergualin, FTY720 represent a collection of suitable agents which may be used in combination with at least one compound of the general formulas (I) to (VIII) and/or pharmaceutically active salts thereof.

The aromatic guanylhydrazone compounds of the general formulas (I) and (II) are basic and form pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphersulfonic acid, china acid, mandelic acid, o-methylmandelic acid, hydrogen-benzenesulfonic acid, picric acid, adipic acid, d-o-tolyltartraric acid, tartronic acid, α-toluic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

The free base forms may be regenerated by treating the salt with a suitable dilute aqueous base solution such as dilute aqueous sodium hydroxide, potassium carbonate, ammonia and sodium bicarbonate. The free base forms differ from their corresponding salt forms somewhat in certain physical properties, such as solubility in polar solvents, but the salts are otherwise equivalent to their corresponding free base forms for purposes of this invention.

A still further aspect of the present invention relates to pharmaceutical compositions comprising at least one compound according to the invention and/or pharmaceutically acceptable salts thereof as an active ingredient and a pharmaceutically acceptable carrier, excipient, adjuvent and/or diluent.

The compounds of the general formulas (I) to (VIII) can also be administered in form of their pharmaceutically active salts optionally using substantially nontoxic pharmaceutically acceptable carrier, excipients, adjuvents or diluents. The medications of the present invention are prepared in a conventional solid or liquid carrier or diluent and a conventional pharmaceutically-made adjuvant at suitable dosage level in a known way. The preferred preparations are in administratable form which is suitable for oral application. These administratable forms, for example, include pills, tablets, film tablets, coated tablets, capsules, powders and deposits. Other than oral adminisratable forms are also possible. The inventive guanylhydrazone compounds of the general formulas (I) and/or (II) or pharmaceutical preparations containing said compounds may be administed by any appropriate means, including but not limited to injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrally, intracutanly, intravaginally, intravasally, intranasally, intrabuccally, percutanly, sublingually, or any other means available within the pharmaceutical arts.

Within the disclosed methods the pharmaceutical compositions of the present invention, containing at least one aromatic guanylhydrazone compound according to the invention or pharmaceutically acceptable salts thereof as an active ingredient will typically be administered in admixture with suitable carrier materials suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.
Suitable binders include starch, gelatin, natural sugars, corn sweeteners, natural and synthetic gums such as acacia, sodium alginate, carboxymethyl-cellulose, polyethylene glycol and waxes. Among the lubricants there may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below. Additionally, the compositions of the present invention may be formulated in sustained release form to provide the rate controlled release of any one or more of the components or active ingredients to optimize the therapeutic effects, i.e. antihistaminic activity and the like.
Suitable dosage forms for sustained release include layered tablets containing layers of varying disintegration rates or controlled release polymeric matrices impregnated with the active components and shaped in tablet form or capsules containing such impregnated or encapsulated porous polymeric matrices.
Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.
For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidify.
Also included are solid form preparations which are intended to be converted, shortly before use, to liquid form preparations for either oral or parenteral administration. Such liquid forms include solutions, suspensions and emulsions.
The aromatic guanylhydrazone compounds of the present invention may also be deliverable transdermally. The transdermal compositions may take the form of creams, lotions, aerosols and/or emulsions and can be included in a transdermal patch of the matrix or reservoir type as are conventional in the art for this purpose.
The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.
Tablet means compressed or molded solid dosage form containing the active ingredients with suitable diluents. The tablet can be prepared by compression of mixtures or granulations obtained by wet granulation, dry granulation or by compaction well known to a person skilled in the art.
Oral gels refers to the active ingredients dispersed or solubilized in a hydrophillic semi-solid matrix. Powders for constitution refers to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluent in the composition can range from about 5 to about 95% by weight of the total composition, preferably from about 25 to about 75%, more preferably from about 30 to about 60% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and crosslinked microcrystalline celluloses such as sodium croscarmellose, alginates such as alginic acid and sodium alginate, clays such as bentonites, and effervescent mixtures. The amount of disintegrant in the composition can range from about 2 to about 20% by weight of the composition, more preferably from about 5 to about 10% by weight.
Binders characterize substances that bind or "glue" powders together and make them cohesive by forming granules, thus serving as the "adhesive" in the formulation. Binders add cohesive strength already available in the diluent or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 2 to about 20% by weight of the composition, more preferably from about 3 to about 10% by weight, even more preferably from about 3 to about 6% by weight. Lubricant refers to a substance added to the dosage form to enable the tablet, granules, etc. after It has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'l-leucine. Lubricants are usually added at the very last step before compression, since they must be present on the surfaces of the granules and in between them and the parts of the tablet press. The amount of lubricant in the composition can range from about 0.2 to about 5% by weight of the composition, preferably from about 0.5 to about 2%, more preferably from about 0.3 to about 1.5% by weight.
Glidents are materials that prevent caking and improve the flow characteristics of granulations, so that flow is smooth and uniform. Suitable glidents include silicon dioxide and talc. The amount of glident in the composition can range from about 0.1% to about 5% by weight of the total composition, preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the colouring agent can vary from about 0.1 to about 5% by weight of the composition, preferably from about 0.1 to about 1%.

Techniques for the formulation and administration of the aromatic guanylhydrazone compounds of the present invention may be found in "Remington's Pharmaceutical Sciences" Mack Publishing Co., Easton PA. A suitable composition comprising at least one compound of the invention may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

### EXAMPLE:

### Cell Culture Medium

Cells were cultured using a standard medium (referred to as 1% human plasma medium), which consisted of RPMI 1640 (BioWhittaker) supplemented with glutamine (300 µg/ml) (BioWhittaker), penicillin/streptomycin (20 µg/ml), 10 mM Hepes, pH 7.5 (Sigma-Aldrich) and 1% heat-inactivated (56°C; 30 min) human plasma from a single donor.

### Generation of DC

Peripheral Blood Mononuclear Cellss (5 x 10⁷) were isolated from buffy coats by sedimentation in Ficoll-hypaque (Amersham) and seeded onto IgG-coated (10 µg/ml -globulin from Cohn fraction; Sigma-Aldrich) 100 mm-culture dishes and incubated at 37°C in 5% CO₂. After 1 and 7 h of incubation, nonadherent cell fractions were harvested, and the remaining adherent cells (predominantly monocytes) were further cultured in 1% human plasma medium supplemented with the cytokines GM-CSF (800 U/ml) and IL-4 (1,000 U/ml). Fresh medium (5 ml) containing 4,000 U GM-CSF and 5,000 U IL-4 was added to the culture dish at day 3 of this incubation period. On day 4 or 5, nonadherent cells were collected, counted, and transferred into new dishes at a density of 0.3-0.5 x 10⁵ cells/ml. For final DC maturation, 1% human plasma medium was supplemented with TNF- (25 ng/ml), prostaglandin E₂ (PGE₂; 1 ng/ml), GM-CSF (400 U/ml), and IL-4 (500 U/ml).

### Cytokines

Recombinant human (rh)GM-CSFwas obtained from Novartis Research Institute, rhIL-4 from Genzyme, rhTNF- from Boehringer, and PGE₂ from Cayman Chemical.

### Experimental Readouts

a) Uptake of dextran as a measure of dendritic cell activity
b) Expression of cell surface markers as a measure for dendritic cell phenotype. For flow cytometry analyses, mAbs recognizing the following antigens were used: CD83 (Immunotech), CD86, CD32, MHC class II (Becton Dickinson). The isotype controls IgG1a and IgG2b were obtained from Becton Dickinson and were run in parallel. Cell populations were analyzed on a FACScan™ (Becton Dickinson) according to the manufacturer's recommendations.
c) Antigen presentation to the T-lymphocytes in an allogeneic mixed lymphocyte reaction as a measure for the immunocompetence of the dendritic cells.

### Results

a) After 5 days in culture under GM-CSF (800 U/ml) and IL-4 (1,000 U/ml) conditions, monocyte-derived dendritic cells showed a marked capability of FITC-labeled dextran uptake as compared to day 1 of culture. In sharp contrast, cells trated for 5 days with 2 micromolar concentrations of compound No. 4 showed only a marginal capacity of dextran uptake.
b) After 5 days in culture under GM-CSF (800 U/ml) and IL-4 (1,000 U/ml) conditions, monocyte-derived dendritic cells showed a strong increase of CD32, HLA class II, and CD83 surface markers when compared to day 1 of culture. In sharp contrast, cells trated for 5 days with 2 micromolar concentrations of compound No. 4 showed only very low levels of CD32, HLA class II and CD83 surface markers when compared to day 1 of culture treatment.
c) The most distinctive functional characteristic of DCs is their ability to induce a potent T cell response in an allogeneic Mixed Lymphocyte Reaction. After 5 days in culture under GM-CSF (800 U/ml) and IL-4 (1,000 U/ml) conditions, monocyte-derived dendritic cells displayed strong T cell proliferation induction rates. In sharp contrast, cells trated for 5 days with 2 micromolar concentrations of compound No. 4 clearly impaired the ability of DCs to induce a significant T cell response in the MLR.

All these data clearly point to the fact that compound 4 prevents the maturation, activation and biological function of human dendritic cells as the most potent antigen presenting and immunocompetent cell type. Therefore the compounds of the present invention are useful for the prevention of graft rejection in organ transplantation as a consequence of being able to pacify dendritic cells.

## Claims

1. Use of a compound of the formula I or a pharmaceutically acceptable salt thereof for the manufacture of a medicament for the inhibition of a CD83 functionally dependent process in an individual, wherein the formula I is defined as: 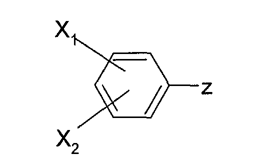 and the characters in the formula have the following meanings:
X_{1,}X₁'_{,}X₁" are - CR¹=N-NH-C(NH)NH₂;
X₂,X₂',X₂" are -CR²=N-NH-C(NH)NH₂, -H, -OCH₃
Z is -H, -NH₂, -CH=CH-COOH, -C₆H₅ (phenyl), -C₅NH₄ (pyridyl); -COOB, -OB, -OCOB, -C₆H₄-B, -C₅NH₃-B; -CR=N-NH-C(NH)NH₂, ; -A-B; -A-C₆H₅, -A-OB, -A-(CHR)ₙ-B, -A-(CHR)ₙ- A'-B, -A-(CHR)ₙ-Q-(CHR')ₘ-B, -A-(CHR)ₙ-Q-(CHR')ₘ- A'-B
A, A', A" are -NHCO-, -CONH-, -NH;-
R, R¹, R², R', R" are -H, C₁ - C₃- alkyl;
B is 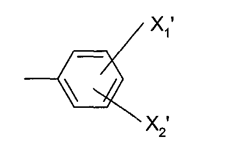 B' is 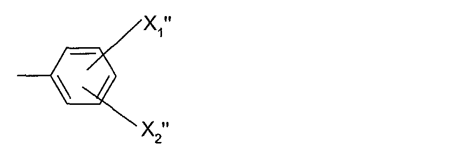
Q is 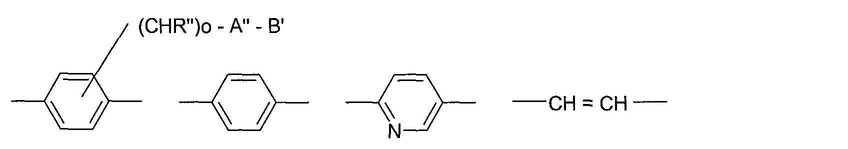
n, m, o are independently from each other integers from 0 to 10.

2. Use according to claim 1, wherein said compound of formula I is selected from the group consisting of following sub-formulas II - VIII, wherein the characters in the formulas have the meaning as indicated in claim 1.

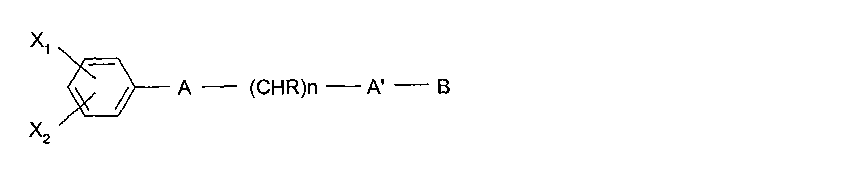

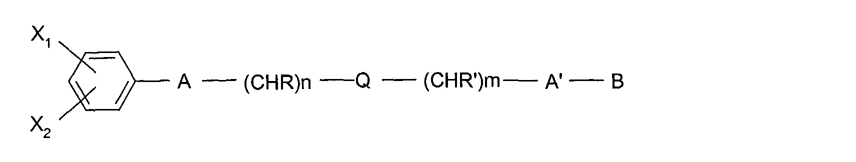

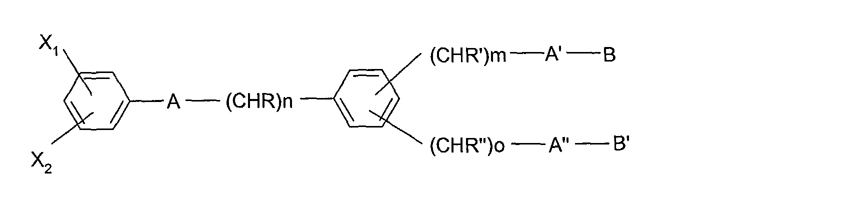

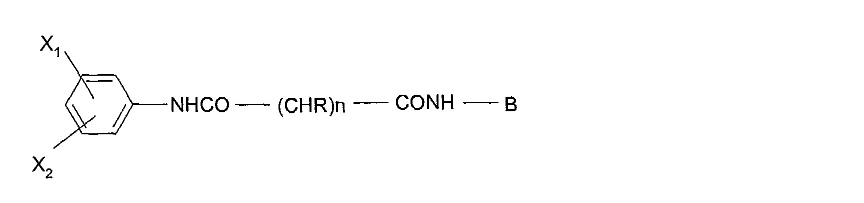

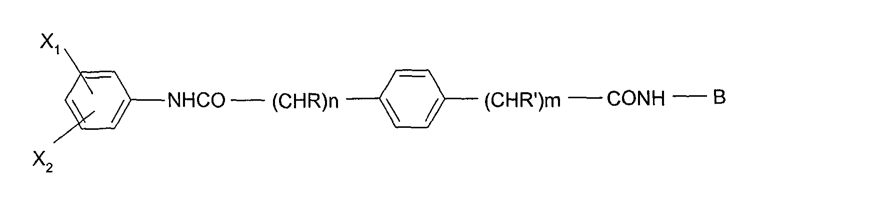

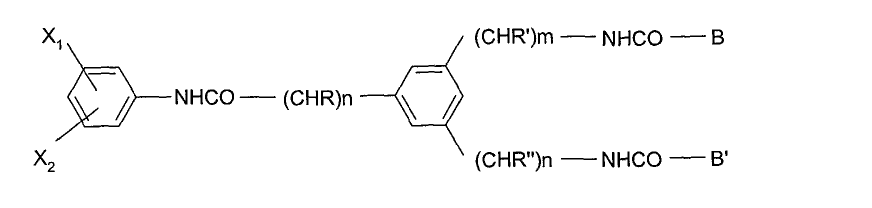

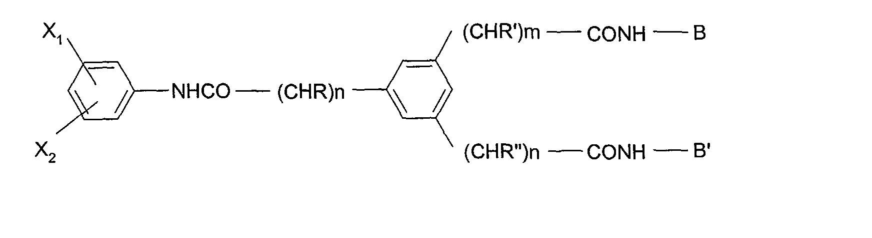

3. Use according to claim 1 or 2, wherein compounds are selected where A and A' are different.

4. Use according to claim 1, 2 or 3, wherein said inhibition is directed to the nucleocytoplasmic translocation of CD83 mRNA molecules.

5. Use according to any of the claims 1 to 4, wherein said inhibition is directed to the expression of CD83 protein.

6. Use according to claim 5, wherein the expression of CD83 on dentritic cells is inhibited.

7. Use according to claim 5 or 6, wherein said inhibition is directed to the maturation of dentritic cells.

8. Use according to any of the claims 1 to 7, for the treatment and / or prevention of graft rejection during or following organ transplantation.

9. Use according to claim 8, wherein the organ suitable for said transplantation is selected from the group: kidney, liver, heart, pancreas, lung and bone marrow.

10. Use according to claim 8 or 9, wherein said organ transplantation comprises treatment of cells of said organ *ex vivo* with any of the compounds of the formulas I - VIII.

11. Use according to claim 10, wherein said cells are the complete or parts of the bone marrow cells of said individual.

12. Use according to claim 10 or 11, wherein the effective dosage of any of said compounds corresponds to a concentration in the range of 0.01 - 10 µM, preferably 0.1 -5 µM.

13. Use according to any of the claims 8 to 12, wherein said treatment is achieved in combination with further medicaments used in or after organ transplantation.

14. Use according to claim 13, wherein the effective dosage of said further medicament is lower than when administered alone in monotherapy.

15. Use according to claim 13 or 14, wherein said further medicament is selected from the group consisting of: mycophenolat mofetil, brequinar, cyclosporine, tacrolimus, sirolimus, spergualin, FTY720.

16. A pharmaceutical composition comprising one or more compounds of the formulas I to VIII or a pharmaceutically acceptable salt thereof, a further different medicament suitable in or after organ transplantation, and, optionally, a pharmaceutically acceptable carrier, excipient or diluent.

17. A pharmaceutical composition of claim 16, wherein said further different medicament is selected from the group consisting of: mycophenolat mofetil, brequinar, cyclosporine, tacrolimus, sirolimus, spergualin, FTY720.

18. A pharmaceutical kit comprising a first package containing a compound of any of the formulas I to VIII, as defined in any of the preceding claims, and a second package containing a compound selected from the group consisting of: mycophenolat mofetil, brequinar, cyclosporine, tacrolimus, sirolimus, spergualin, FTY720.

19. Method of inhibiting of a CD83 dependent process in an individual comprising administering to said individual in an effective dosage a compound of formula I or one of its sub-formulas II to VIII, wherein the formulas are defined as: 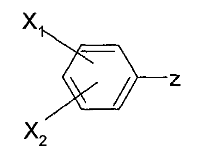 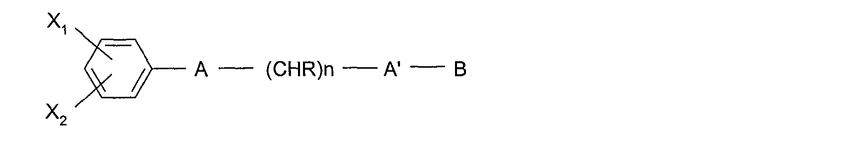 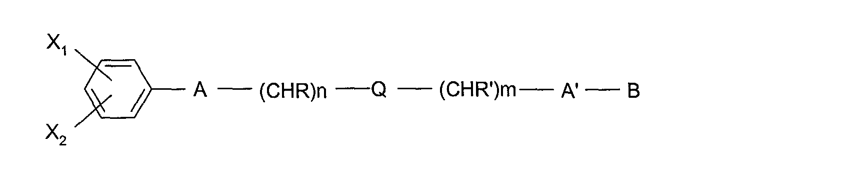 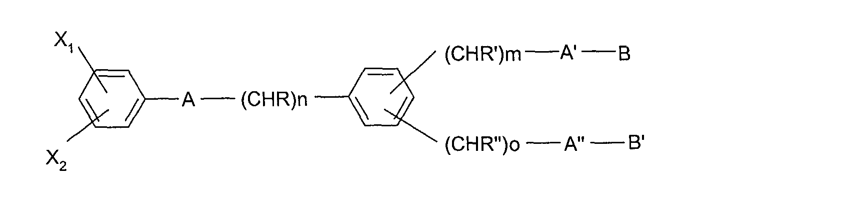 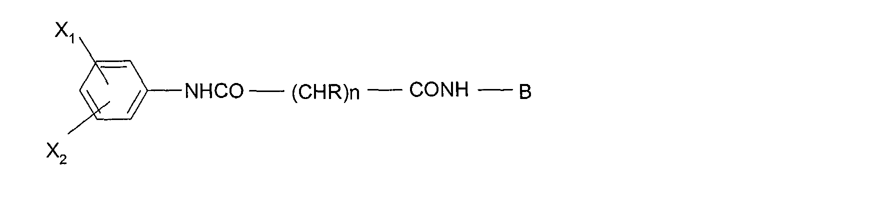 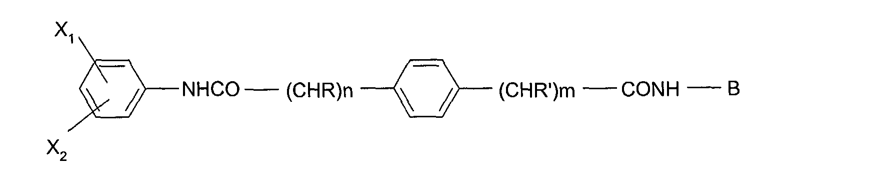 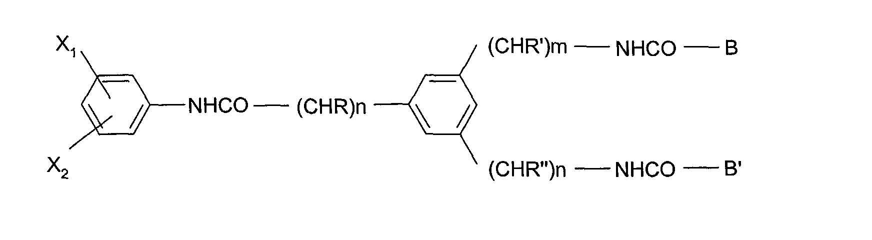 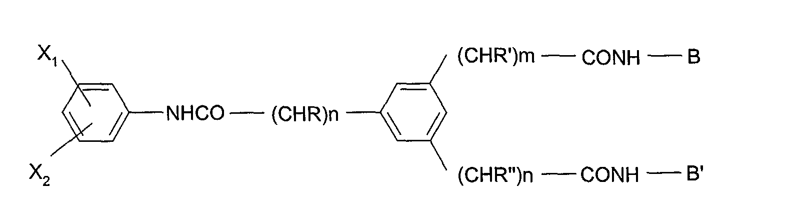 and the characters in the formulas have the following meanings:
X₁, X₁', X₁" are - CR¹ =N-NH-C(NH)NH₂;
X₂, X₂', X₂" are -CR²=N-NH-C(NH)NH₂, -H, -OCH₃
Z is -H, -NH₂, -CH=CH-COOH, -C₆H₅ (phenyl), -C₅NH₄ (pyridyl); -COOB, -OB, -OCOB, -C₆H₄-B, -C₅NH₃-B; -CR=N-NH-C(NH)NH₂, ; -A-B; -A-C₆H₅, -A-OB, -A-(CHR)ₙ-B, -A-(CHR)ₙ- A'-B, -A-(CHR)ₙ-Q-(CHR')ₘ-B, -A-(CHR)ₙ-Q-(CHR)ₘ- A'-B
A, A', A" are -NHCO-, -CONH-, -NH;-
R¹, R², R', R" are -H, C₁ - C₃- alkyl;
B is 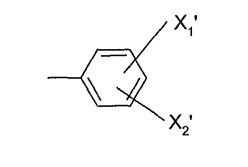 B' is 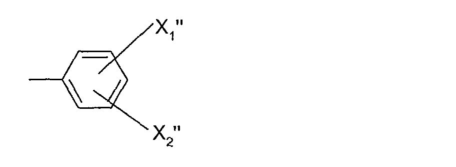
Q is 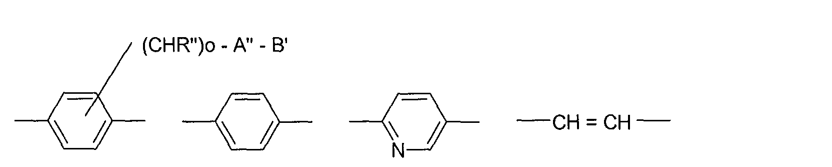
n, m, o are independently from each other integers from 0 to 10.

20. A method of claim 19, wherein compounds are selected where A and A' are different.

21. A method according to claim 19 or 20, wherein said inhibition is directed to the nucleocytoplasmic translocation of CD83 mRNA molecules.

22. A method of claim 19, 20 or 21, wherein said inhibition is directed to the expression of CD83 protein.

23. A method according to claim 22, wherein the expression of CD83 on dentritic cells is inhibited.

24. A method of claim 22 or 23, wherein said inhibition is directed to the maturation of dentritic cells.

25. A method for the treatment and / or prevention of graft rejection during or following organ transplantation comprising administering to an individual in an effective dosage a compound of any of the formulas I to VIII, as defined in any of the preceding claims.

26. A method for the treatment and / or prevention of graft rejection during or following organ transplantation of an individual, comprising isolating diseased cells from said organ and treating them ex vivo with an effective amount of a compound of the formulas I to VIII.

27. A method of claim 26, wherein said diseased cells are the complete or parts of the bone marrow cells of said individual.

28. A method of claim 27, wherein said effective dosage of the compound corresponds to a concentration in the range of 0.01 - 10 µM, preferably 0.1 - 5 µM.

29. A method according to claim 27 or 28, wherein a further medicament is administered to the individual which is used in or after organ transplantation and is different from those compounds as specified in any of the formulas I to VIII.

30. A method according to claim 29, wherein sad further medicament is administered in an effective dosage which is lower than when administered alone in a monotherapy.

31. A method according to claim 29 or 30, wherein said further medicament is selected from the group consisting of: mycophenolat mofetil, brequinar, cyclosporine, tacrolimus, sirolimus, spergualin, FTY720.
